# EUROPEAN PATENT APPLICATION

(11) **EP 1 908 757 A1**
(43) Date of publication of application: **09.04.2008**
(21) Application number: 06767927.4
(22) Date of filing: 06.07.2006
(51) Int. Cl.: C07D 309/10, A61K 31/351, A61P 3/10, C07C 215/90

(54) **CHOLINE SALT CRYSTAL OF AZULENE COMPOUND**

(30) Priority: 07.07.2005 JP 2005198973
(71) Applicant: Astellas Pharma Inc., Tokyo 103-8411 (JP); Kotobuki Pharmaceutical Co., Ltd., Hanishina-gun, Nagano 389-0697 (JP)
(72) Inventor: IMAMURA, Masakazu, honcho, 2-chome, Chuo-ku, Tokyo 103-8411 (JP); SUZUKI, Takayuki, honcho 2-chome, Chuo-ku, Tokyo 103-8411 (JP); MURAKAMI, Takeshi, honcho 2-chome, Chuo-ku, Tokyo 103-8411 (JP); NAKANISHI, Keita, honcho 2-chome, Chuo-ku, Tokyo 103-8411 (JP); UEBAYASHI, Hiroshi, honcho 2-chome, Chuo-ku, Tokyo 103-8411 (JP); NAKAMURA, Haruka, honcho 2-chome, Chuo-ku, Tokyo 103-8411 (JP); YUDA, Masamichi, honcho 2-chome, Chuo-ku, Tokyo 103-8411 (JP); AMENOMIYA, Naoko, honcho 2-chome, Chuo-ku, Tokyo 103-8411 (JP); AWAMURA, Yuuji, honcho 2-chome, Chuo-ku, Tokyo 103-8411 (JP); TOMIYAMA, Hiroshi, Sakaki-machi, Hanishina-gun, Nagano 389-0697 (JP)
(74) Representative: HOFFMANN EITLE
(86) International application number: PCT/JP2006/313468
(87) International publication number: WO 2007/007628

(57) **Abstract**

A choline salt crystal of (1S)-1,5-anhydro-1-[5-(azulen-2-ylmethyl)-2-hydroxyphenyl]-D-glucitol which shows an endothermic peak at 194 to 198°C as measured by differential scanning calorimetry (DSC analysis) and shows main peaks at about 2θ (°) 5.58, 14.72, 16.80, 17.82, 21.02, and 22.46 as measured by X-ray powder diffraction. Thus, a crystal of an azulene compound can be produced which is in a single crystal form, has a constant quality, can be produced with good reproducibility, can be provided stably as a crystal of an drug substance for use in the preparation of a pharmaceutical and is excellent in storage stability.

## Description

### TECHNICAL FIELD

The present invention relates to a choline salt, a choline salt crystal, and a choline salt hydrate crystal of (1S)-1,5-anhydro-1-[5-(azulen-2-ylmethyl)-2-hydroxyphenyl]-D-glucitol (hereinafter referred to from time to time as "azulene compound A" or simply "compound A"). More particularly, the present invention relates to a choline salt, a choline salt crystal, and a choline salt hydrate crystal of azulene compound A obtainable with excellent reproducibility as crystals as a single crystal form having a constant quality, thus being stably available as a crystal of a drug substance used for preparing pharmaceuticals, and having excellent storage stability, and to a pharmaceutical composition particularly useful as a diabetes treating agent.

### BACKGROUND ART

The inventors of the present invention previously disclosed that (1S)-1,5-anhydro-1-[5-(azulen-2-ylmethyl)-2-hydroxyphenyl]-D-glucitol (azulene compound A) is a useful compound as a pharmaceutical, particularly as a Na⁺-glucose cotransporter inhibitor, for treating and preventing diabetes, such as insulin-dependent diabetes mellitus (type 1 diabetes) and noninsulin-dependent diabetes mellitus (type 2 diabetes), insulin resistance diseases, and various diabetes-associated diseases including obesity (WO 2004/013118 (Patent Document 1), see Example 75).

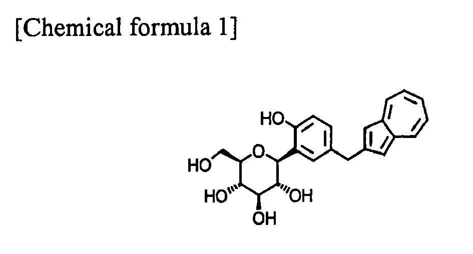

### DISCLOSURE OF THE INVENTION

Although Patent Document 1 describes a free-form azulene compound A, there are no specific descriptions of a salt of the compound A. As a result of extensive studies on the free-form azulene compound A described in the Patent Document 1, the inventors have confirmed that there are two types of hydrate crystals and five types of anhydride crystals. The crystal form of the free-form compound A is variable, and it is technically difficult to obtain target crystals as a single crystal form in the preparation of a raw pharmaceutical compound with good reproducibility. Therefore, it is technically difficult to stably supply crystals of a raw pharmaceutical compound with a constant quality, and it is costwise extremely difficult to stably supply the crystals of the raw pharmaceutical compound. Accordingly, it has been impossible to use the free-form compound A as the raw compound in the preparation of a pharmaceutical in practice.

Next, as a result of extensive studies of Na salt crystals, K salt crystals, Li salt crystals, and Ca salt crystals which are commonly used as pharmaceuticals and a drug substance, it was found that it is extremely difficult to stably supply Na salt crystals, K salt crystals, and Li salt crystals with a constant quality, since these salt crystals change their forms by releasing volatile components at a low temperature. In addition, since the crystals of 1/2 Ca salt are obtained only in a form combined with dimethylformamide (DMF), the problems of toxicity due to DMF are unavoidable.

The present invention has been achieved in order to solve these problems and has an object of providing crystals of azulene compound A obtainable with excellent reproducibility as crystals as a single crystal form having a constant quality, having a high possibility of being stably supplied as a crystal of a drug substance used for preparing pharmaceuticals at a reasonable cost, and having excellent storage stability.

In order to attain the above-mentioned object, the inventors of the present invention have conducted extensive studies on a choline [(CH₃)₃N⁺CH₂CH₂OH] salt which is not commonly used as a pharmaceutical. As a result, the inventors have found that a choline salt of compound A can be obtained with excellent reproducibility as crystals as a single crystal form having a constant quality, can stably be supplied as a crystal of a drug substance used for preparing pharmaceuticals, and has excellent storage stability and that, although a choline salt of compound A produces a hydrate crystal of the choline salt of compound A when processed under high humidity conditions, the hydrate crystal can also be useful as a drug substance for preparing pharmaceuticals. These findings have led to completion of the present invention. That is, in order to attain the above object, the following choline salts, choline salt crystals, and choline salt hydrate crystals of azulene compound A, and a pharmaceutical composition particularly suitable as a diabetes treating agent are provided according to the present invention.

[1] A choline salt of
   (1S)-1,5-anhydro-1 -[5-(azulen-2-ylmethyl)-2-hydroxyphenyl]-D-glucitol.

[2] A choline salt crystal of
   (1S)-1,5-anhydro-1-[5-(azulen-2-ylmethyl)-2-hydroxyphenyl]-D-glucitol having an endothermic peak at 194 to 198°C measured by differential scanning calorimetry analysis (DSC analysis).

[3] A choline salt crystal of
   (1S)-1,5-anhydro-1-[5-(azulen-2-ylmethyl)-2-hydroxyphenyl]-D-glucitol having main peaks at about 2θ (°) 5.58, 14.72, 16.80, 17.82, 21.02, and 22.46 measured by X-ray powder diffraction.

[4] A choline salt crystal of
   (1S)-1,5-anhydro-1-[5-(azulen-2-ylmethyl)-2-hydroxyphenyl]-D-glucitolhaving an endothermic peak at 194 to 198°C measured by differential scanning calorimetry analysis (DSC analysis) and main peaks at about 2θ (°) 5.58, 14.72, 16.80, 17.82, 21.02, and 22.46 measured by X-ray powder diffraction.

**[5]** A choline salt hydrate crystal of
   (1S)-1,5-anhydro-1-[5-(azulen-2-ylmethyl)-2-hydroxyphenyl]-D-glucitol having a broad endothermic peak at about 78°C and an endothermic peak at 195 to 199°C measured by differential scanning calorimetry analysis (DSC analysis).

[6] A choline salt hydrate crystal of
   (1S)-1,5-anhydro-1-[5-(azulen-2-ylmethyl)-2-hydroxyphenyl]-D-glucitol having main peaks at about 2θ (°) 5.66, 17.08, 17.66, 19.02, 19.58, and 22.14 measured by X-ray powder diffraction.

[7] A choline salt hydrate crystal of
   (1S)-1,5-anhydro-1-[5-(azulen-2-ylmethyl)-2-hydroxyphenyl]-D-glucitol having a broad endothermic peak at about 78°C and an endothermic peak at 195 to 199°C measured by differential scanning calorimetry analysis (DSC analysis) and main peaks at about 2θ (°) 5.66, 17.08, 17.66, 19.02, 19.58, and 22.14 measured by X-ray powder diffraction.

[8] A pharmaceutical composition comprising the choline salt crystal according to [1], the choline salt crystal according to any one of [2] to [4], or the choline salt hydrate crystal according to any one of [5] to [7] as an effective ingredient.

[9] The pharmaceutical composition according to [8], further comprising a pharmaceutically acceptable excipient.

[10] The pharmaceutical composition according to [8] or [9] which is a diabetes treating agent.

A choline salt, a choline salt crystal, and a choline salt hydrate crystal of azulene compound A obtainable with excellent reproducibility as crystals as a single crystal form having a constant quality, thus being stably supplied as a crystal of a drug substance used for preparing pharmaceuticals, and having excellent storage stability, and a pharmaceutical composition particularly useful as a diabetes treating agent are provided according to the present invention.

### BRIEF DESCRIPTION OF THE DRAWINGS

[Fig. 1] Fig. 1 is a differential scanning calorimetry analysis chart (DSC analysis chart) of crystals of [(2-hydroxyethyl)trimethylammonium 4-(azulen-2-ylmethyl)-2-β-D-glucopyranosylphenolate] (choline salt of azulene compound A).
[Fig. 2] Fig. 2 is a X-ray powder diffraction chart of crystals of [(2-hydroxyethyl)trimethylammonium 4-(azulen-2-ylmethyl)-2-β-D-glucopyranosylphenolate] (choline salt of azulene compound A).
[Fig. 3] Fig. 3 is a differential scanning calorimetry analysis chart (DSC analysis chart) of a hydrate of [(2-hydroxyethyl)trimethylammonium 4-(azulen-2-ylmethyl)-2-β-D-glucopyranosylphenolate] (choline salt hydrate of azulene compound A).
[Fig. 4] Fig. 4 is a X-ray powder diffraction chart of crystals of [(2-hydroxyethyl)trimethylammonium 4-(azulen-2-ylmethyl)-2-β-D-glucopyranosylphenolate hydrrate] (choline salt hydrate of azulene compound A).
[Fig. 5] Fig. 5 is a differential scanning calorimetry analysis chart (DSC analysis chart) of crystals of [sodium 4-(azulen-2-ylmethyl)-2-β-D-glucopyranosylphenolate] (sodium salt of azulene compound A).
[Fig. 6] Fig. 6 is a X-ray powder diffraction chart of crystals of [sodium 4-(azulen-2-ylmethyl)-2-β-D-glucopyranosylphenolate] (sodium salt of azulene compound A).
[Fig. 7] Fig. 7 is a differential scanning calorimetry analysis chart (DSC analysis chart) of crystals of [potassium 4-(azulen-2-ylmethyl)-2-β-D-glucopyranosylphenolate] (potassium salt of azulene compound A).
[Fig. 8] Fig. 8 is a X-ray powder diffraction chart of crystals of [potassium 4-(azulen-2-ylmethyl)-2-β-D-glucopyranosylphenolate] (potassium salt of azulene compound A).
[Fig. 9] Fig. 9 is a differential scanning calorimetry analysis chart (DSC analysis chart) of crystals of [lithium 4-(azulen-2-ylmethyl)-2-β-D-glucopyranosylphenolate] (lithium salt of azulene compound A).
[Fig. 10] Fig. 10 is a X-ray powder diffraction chart of crystals of [lithium 4-(azulen-2-ylmethyl)-2-β-D-glucopyranosylphenolate] (lithium salt of azulene compound A).
[Fig. 11] Fig. 11 is a differential scanning calorimetry analysis chart (DSC analysis chart) of crystals of [hemicalcium 4-(azulen-2-ylmethyl)-2-β-D-glucopyranosylphenolate] (1/2 calcium salt of azulene compound A).
[Fig. 12] Fig. 12 is a X-ray powder diffraction chart of crystals of [hemicalcium 4-(azulen-2-ylmethyl)-2-β-D-glucopyranosylphenolate] (1/2 calcium salt of azulene compound A).
[Fig. 13] Fig. 13 is a differential scanning calorimetry analysis chart (DSC analysis chart) of [(1S)-1,5-anhydro-1-[5-(azulen-2-ylmethyl)-2-hydroxyphenyl]-D-glucitol hydrate crystal-1] (hydrate crystal-1 of azulene compound A).
[Fig. 14] Fig. 14 is a X-ray powder diffraction chart of [(1S)-1,5-anhydro-1-[5-(azulen-2-ylmethyl)-2-hydroxyphenyl]-D-glucitol hydrate crystal-1] (hydrate crystal-1 of azulene compound A).
[Fig. 15] Fig. 15 is a differential scanning calorimetry analysis chart (DSC analysis chart) of [(1S)-1,5-anhydro-1-[5-(azulen-2-ylmethyl)-2-hydroxyphenyl]-D-glucitol hydrate crystal-2] (hydrate crystal-2 of azulene compound A).
[Fig. 16] Fig. 16 is a X-ray powder diffraction chart of [(1S)-1,5-anhydro-1-[5-(azulen-2-ylmethyl)-2-hydroxyphenyl]-D-glucitol hydrate crystal-2] (hydrate crystal-2 of azulene compound A).
[Fig. 17] Fig. 17 is a X-ray powder diffraction (heating X-ray powder) chart of [(1S)-1,5-anhydro-1-[5-(azulen-2-ylmethyl)-2-hydroxyphenyl]-D-glucitol anhydride crystal-1] (anhydride crystal-1 of azulene compound A).
[Fig. 18] Fig. 18 is a X-ray powder diffraction (heating X-ray powder) chart of [(1S)-1,5-anhydro-1-[5-(azulen-2-ylmethyl)-2-hydroxyphenyl]-D-glucitol anhydride crystal-2] (anhydride crystal-2 of azulene compound A).
[Fig. 19] Fig. 19 is a differential scanning calorimetry analysis chart (DSC analysis chart) of [(1S)-1,5-anhydro-1-[5-(azulen-2-ylmethyl)-2-hydroxyphenyl]-D-glucitol anhydride crystal-3] (anhydride crystal-3 of azulene compound A).
[Fig. 20] Fig. 20 is a X-ray powder diffraction chart of [(1S)-1,5-anhydro-1-[5-(azulen-2-ylmethyl)-2-hydroxyphenyl]-D-glucitol anhydride crystal-3] (anhydride crystal-3 of azulene compound A).
[Fig. 21] Fig. 21 is a differential scanning calorimetry analysis chart (DSC analysis chart) of [(1S)-1,5-anhydro-1-[5-(azulen-2-ylmethyl)-2-hydroxyphenyl]-D-glucitol anhydride crystal-4] (anhydride crystal-4 of azulene compound A).
[Fig. 22] Fig. 22 is a X-ray powder diffraction chart of [(1S)-1,5-anhydro-1-[5-(azulen-2-ylmethyl)-2-hydroxyphenyl]-D-glucitol anhydride crystal-4] (anhydride crystal-4 of azulene compound A).
[Fig. 23] Fig. 23 is a differential scanning calorimetry analysis chart (DSC analysis chart) of [(1S)-1,5-anhydro-1-[5-(azulen-2-ylmethyl)-2-hydroxyphenyl]-D-glucitol anhydride crystal-5] (anhydride crystal-5 of azulene compound A).
[Fig. 24] Fig. 24 is a X-ray powder diffraction chart of [(1S)-1,5-anhydro-1-[5-(azulen-2-ylmethyl)-2-hydroxyphenyl]-D-glucitol anhydride crystal-5] (anhydride crystal-5 of azulene compound A).

### BEST MODE FOR CARRYING OUT THE INVENTION

The best mode for carrying out the present invention will be described below.
A choline salt, a choline salt crystal, and a choline salt hydrate crystal (hereinafter referred to from time to time as "crystals of the invention") of azulene compound A ((1S)-1,5-anhydro-1-[5-(azulen-2-ylmethyl)-2-hydroxyphenyl]-D-glucitol) have chemical structures shown below. As mentioned above, differing from two types of hydrate crystals, five types of anhydride crystals, Na salt crystals, K salt crystals, Li salt crystals, Ca salt crystals of free-form azulene compound A, the crystals of the present invention is obtained with excellent reproducibility as crystals as a single crystal form having a constant quality, can stably be supplied as a crystal of a drug substance used for preparing pharmaceuticals, and have excellent storage stability. The difference of these crystal forms can be distinguished by a differential scanning calorimeter analysis (DSC analysis) and X-ray powder diffraction. "Crystals of the invention" include, in addition to the above-mentioned choline salt crystals and choline salt hydrate crystals, a mixture of choline salt crystals and choline salt hydrate crystals and a mixed crystal of choline salt crystal and choline salt hydrate crystal.

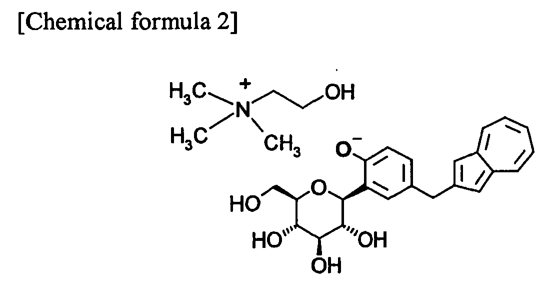

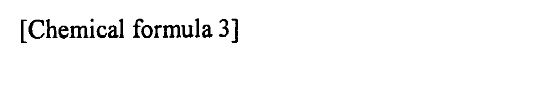

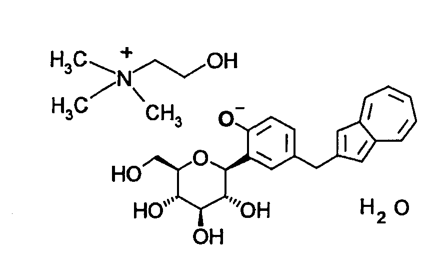

Specifically, among the crystals of the invention, the choline salt crystal has an endothermic peak at 194 to 198°C measured by differential scanning calorimetry (DSC analysis) and/or has main peaks at about 2θ (°) 5.58, 14.72, 16.80, 17.82, 21.02, and 22.46 measured by X-ray powder diffraction, and the choline salt hydrate crystal has a broad endothermic peak at about 78°C and an endothermic peak at 195 to 199°C measured by differential scanning calorimetry (DSC analysis) and/or main peaks at about 2θ (°) 5.66, 17.08, 17.66, 19.02, 19.58, and 22.14 measured by X-ray powder diffraction.

Among the crystals of the invention, the choline salt crystal and the choline salt hydrate crystal are characterized by the diffraction angle (2θ (°)) and the relative intensity measured by X-ray powder diffraction, which are respectively shown in Table 1 and Table 2. Due to the nature of the data obtained by the X-ray powder diffraction, the crystal lattice interval and overall pattern are important in identifying crystals, and the relative intensity, which more or less varies according to the direction of crystal growth, the size of particles, and measuring conditions, should not strictly be construed.

**TABLE 1**

| Diffraction angle | Relative intensity | Diffraction angle | Relative intensity |
|---|---|---|---|
| 5.58 | Strong | 17.82 | Fair |
| 14.72 | Slightly weak | 21.02 | Fair |
| 16.80 | Strong | 22.46 | Fair |

**TABLE 2**

| Diffraction angle | Relative intensity | Diffraction angle | Relative intensity |
|---|---|---|---|
| 5.66 | Strong | 19.02 | Fair |
| 17.08 | Strong | 19.58 | Strong |
| 17.66 | Fair | 22.14 | Strong |

X-ray powder diffraction and differential scanning calorimeter analysis (DSC analysis) were conducted under the following conditions.

### (X-ray powder diffraction)

"MAC Science MXP18TAHF22" equipped with a copper X-ray tube was used under the conditions of a current of 40 mA, a tube voltage of 40 or 200 kV, a sampling width of 0.020°, a scanning rate of 3°/min, wavelength of 1.54056 Å, and measurement angles of diffraction range of (2θ): 3 or 5 to 40°.

### (Differential scanning calorimeter analysis (DSC analysis))

"TA Instrument TA 5000" was used at a temperature from room temperature to 300°C (10°C/min) and a N₂ feed rate of 50 ml/min using an aluminum sampling pan.

### (Method of preparation)

The crystals of the invention can be prepared from the free-form azulene compound A described in Example 75 of Patent Document 1 by a common salt-forming reaction.

The pharmaceutical composition of the present invention contains crystals of the invention and may further comprise a pharmaceutically acceptable excipient, and is particularly useful as a diabetes treating agent.

The pharmaceutical composition containing one or two or more types of the crystals of the invention as effective ingredients can be formed into tablets, powders, subtle granules, granules, capsules, pills, liquid preparations, injections, suppositories, ointments, pasting agents, and the like, using excipients, vehicles, and other additives which are commonly used for preparing pharmaceuticals. These preparations are administered orally or non-orally.

Although a clinical dose of the crystals of the invention for a human is appropriately determined taking into consideration the symptoms, weight, age, sex, and the like of the patient to whom the pharmaceutical is administered, a daily dose to an adult is usually 0.1 to 500 mg per-oral and 0.01 to 100 mg per-nonoral administration. These doses are prescribed to the patient at one time or over several applications.
Since a dose fluctuates according to various conditions, a dose smaller than the above range is sufficient in some cases.

A tablet, a powder, a granule, and the like are used as a solid composition of crystals of the invention for oral administration. In such a solid composition, one or more active compounds are mixed with at least one inert diluent such as lactose, mannitol, glucose, hydroxypropyl cellulose, microcrystalline cellulose, starch, polyvinylpyrrolidone, and magnesium aluminometasilicate. According to a common practice, the composition may contain additives other than the inert diluent. For example, a lubricant such as magnesium stearate, a disintegrator such as cellulose calcium glycolic acid, a stabilizer such as lactose, and a solubilizing agent or a solubilizing adjuvant such as glutamic acid or aspartic acid may be added. As required, the tablets or pills may be provided with a sugar coating such as a coating of sucrose, gelatin, hydroxypropyl cellulose, or hydroxypropyl methylcellulose phthalate, or a film of an enteric or stomach soluble substance.

The liquid composition for oral administration contains a pharmaceutically acceptable emulsifier, solution agent, suspending agent, syrup, elixir, and the like, as well as a common inert diluent such as purified water and ethyl alcohol. In addition to the inert diluents, the composition may contain an assisting agent such as a solubilizing agent, a solubilizing adjuvant, a wetting agent, and a suspending agent, as well as a sweetener, a flavor agent, a perfume, and an antiseptic agent.

The injection preparation to be nonorally administered contains a sterile aqueous or non-aqueous solution agent, a suspending agent, and an emulsifier. As examples of the aqueous solution agent and aqueous diluent of a suspending agent, distilled water for injection and a physiological saline solution can be given. As examples of the non-aqueous solution agent and non-aqueous diluent of a suspending agent, vegetable oils such as propylene glycol, polyethylene glycol, and olive oil; alcohols such as ethyl alcohol; and Polysolvate 80 (commercial name) can be given.

The composition may further contain other additives such as an isotonic agent, an antiseptic agent, a wetting agent, an emulsifier, a dispersant, a stabilizer (for example, lactose), a solubilizing agent, and a solubilizing adjuvant. These additives are sterilized by filtration through a bacteria suspension filter, addition of a disinfectant, or irradiation. A sterile solid composition may be prepared from these additives and dissolved in aseptic water or a sterile solvent for injection prior to use.

### Examples

The present invention will be described in more detail by examples which are not intended to be limiting of the present invention.

### Example 1

### Crystal of [(2-hydroxyethyl)trimethylammonium 4-(azulen-2-ylmethyl)-2-β-D-glucopyranosylphenolate] (choline salt of azulene compound A)

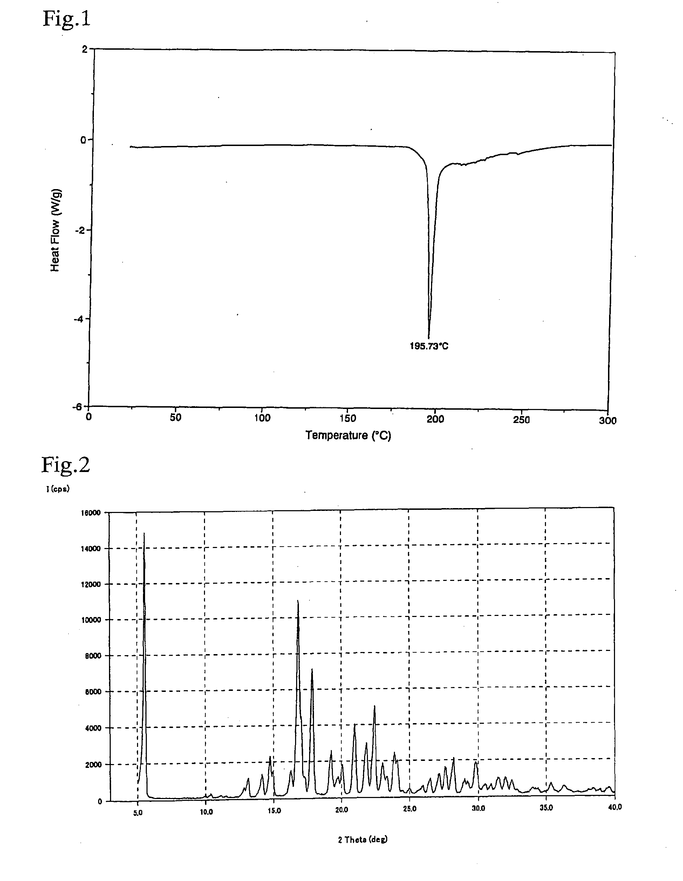

Choline hydroxide (50% aqueous solution) (0.6 ml) was added to a solution of [(1S)-1,5-anhydro-1-[5-(azulen-2-ylmethyl)-2-hydroxyphenyl]-D-glucitol (1.0 g) in methanol (10 ml), and the mixture was stirred at room temperature. The solvent was evaporated under reduced pressure, and co-evaporated with toluene, followed by drying under reduced pressure. After the addition of ethanol (20 ml), the mixture was heated with stirring until the residue was completely dissolved. The mixture was allowed to cool to room temperature. Deposited crystals were collected by filtration, washed with ethanol, and dried at 50°C under reduced pressure. After the addition of ethanol (46 ml) to the resulting solid (1.15 g), the mixture was heated with stirring until the solid was completely dissolved. The mixture was allowed to cool to room temperature. Deposited crystals were collected by filtration, washed with ethanol, and dried at 50°C under reduced pressure to obtain (2-hydroxyethyl)trimethylammonium 4-(azulen-2-ylmethyl)-2-β-D-glucopyranosylphenolate (1.02 g). A differential scanning calorimeter chart (DSC analysis chart) is shown in Fig. 1 and a X-ray powder diffraction chart is shown in Fig. 2.

### Example 2

### Crystal of [(2-hydroxyethyl)trimethylammonium 4-(azulen-2-ylmethyl)-2-β-D-glucopyranosylphenolate hydrate] (choline salt hydrate of azulene compound A)

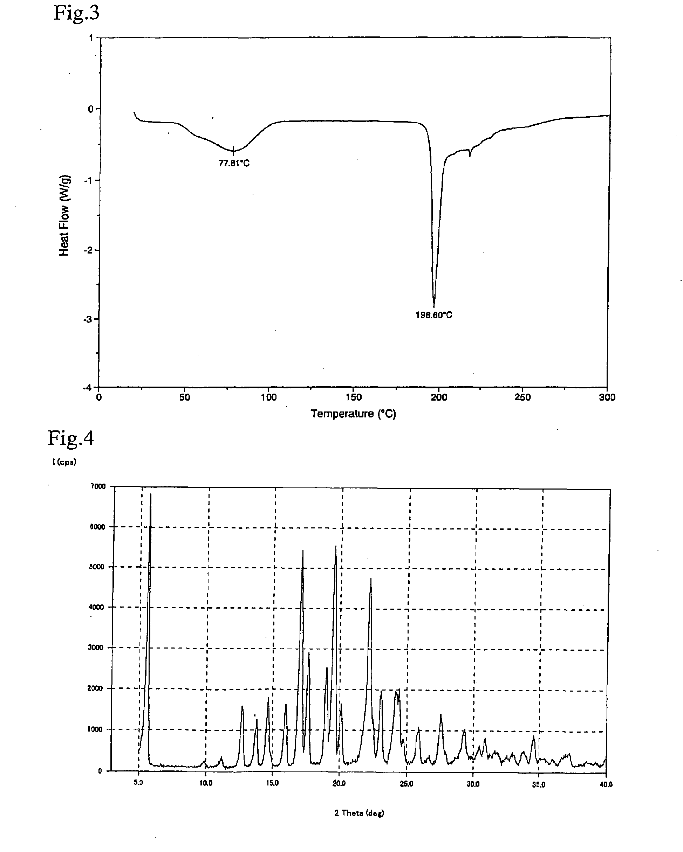

(2-hydroxyethyl)trimethylammonium 4-(azulen-2-ylmethyl)-2-β-D-glucopyranosylphenolate (choline salt) (1.00 g) was preserved for one week in a desiccator in which the relative humidity was adjusted to 93% using potassium nitrate at 25°C to obtain hydrate crystals of (2-hydroxyethyl)trimethylammonium 4-(azulen-2-ylmethyl)-2-β-D-glucopyranosylphenolate (choline salt hydrate) (1.04 g). A differential scanning calorimeter chart (DSC chart) is shown in Fig. 3 and a X-ray powder diffraction chart is shown in Fig. 4.

### Comparative Example 1

### Crystals of [sodium 4-(azulen-2-ylmethyl)-2-p-D-glucopyranosylphenolate] (sodium salt of azulene compound A)

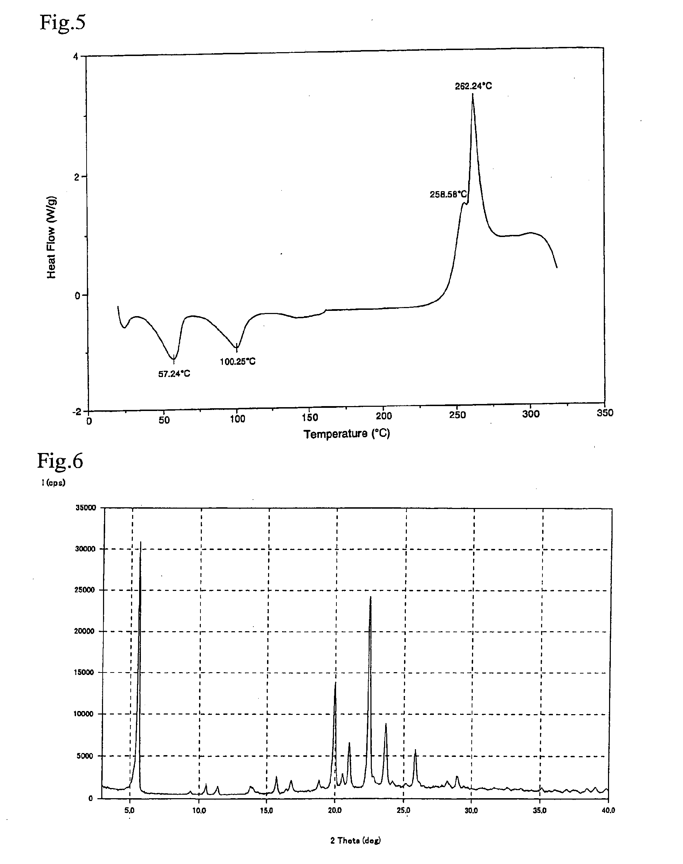

Ethanol (10 ml) and methanol (10 ml) were added to (1S)-1,5-anhydro-1-[5-(azulen-2-ylmethyl)-2-hydroxyphenyl]-D-glucitol (991 mg).
After the addition of a 1M aqueous solution of sodium hydroxide (2.5 ml), the mixture was stirred at room temperature. The solvent was evaporated under reduced pressure and co-evaporated with toluene, followed by azeotropic distillation. The resulting solid was dried at 55°C under reduced pressure. A 5:1 mixture of 2-propanol and water (7.2 ml) was added to the solid (360 mg), and the mixture was heated with stirring until the solid was completely dissolved. After allowing the mixture to cool to room temperature, the deposited crystals were collected by filtration, washed with a 5:1 mixture of 2-propanol and water, and dried at 45°C under reduced pressure to obtain sodium 4-(azulen-2-ylmethyl)-2-β-D-glucopyranosylphenolate (193 mg). Since the resulting crystals change form due to dissociation of volatile components at a low temperature, it was very difficult to stably supply a product with a constant quality. A
differential scanning calorimeter chart (DSC chart) is shown in Fig. 5, and a X-ray powder diffraction chart is shown in Fig. 6.

### Comparative Example 2

### Crystals of [potassium 4-(azulen-2-ylmethyl)-2-β-D-glucopyranosylphenolate] (potassium salt of azulene compound A)

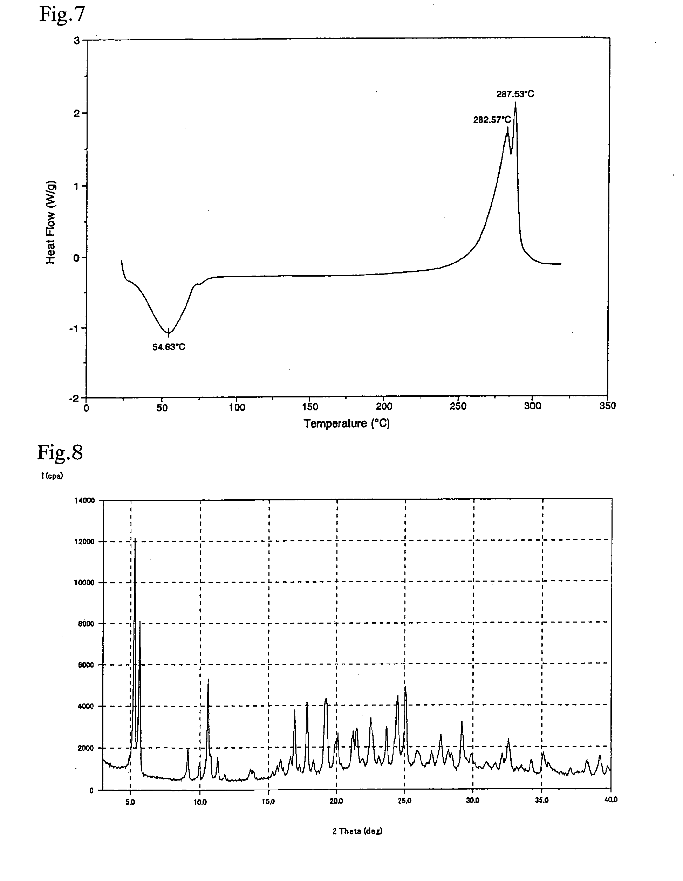

Crystals of potassium 4-(azulen-2-ylmethyl)-2-β-D-glucopyranosylphenolate were obtained in the same manner as in Comparative Example 1 except for using a 1M aqueous solution of potassium hydroxide instead of the 1M aqueous solution of sodium hydroxide. Since the resulting crystals change form due to dissociation of volatile components at a low temperature, it was very difficult to stably supply a product with a constant quality. A differential scanning calorimeter chart (DSC chart) is shown in Fig. 7, and a X-ray powder diffraction chart is shown in Fig. 8.

### Comparative Example 3

### Crystals of [lithium 4-(azulen-2-ylmethyl)-2-β-D-glucopyranosylphenolate] (lithium salt of azulene compound A)

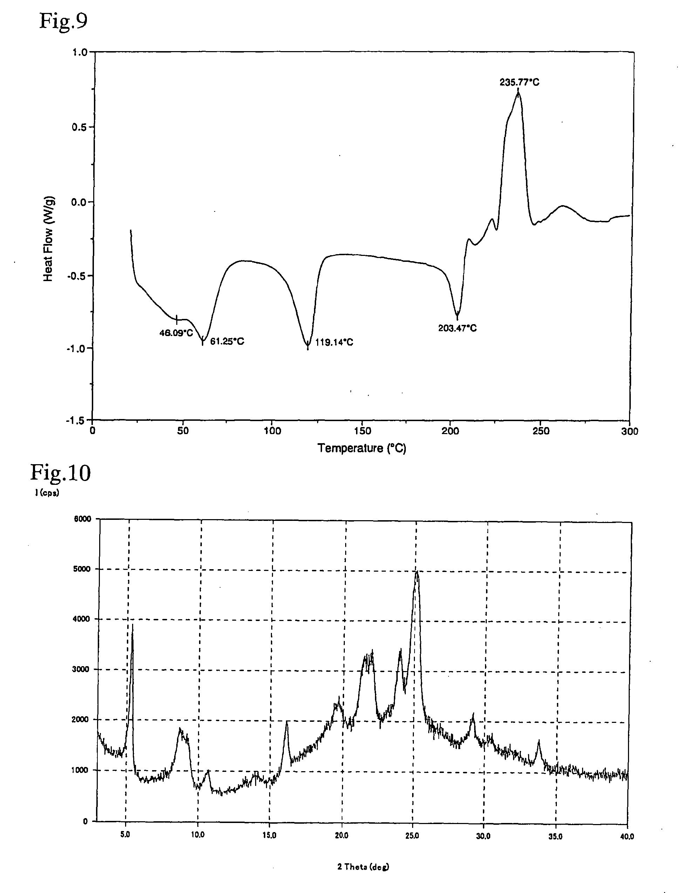

Crystals of lithium 4-(azulen-2-ylmethyl)-2-β-D-glucopyranosylphenolate were obtained in the same manner as in Example 1 except for using a 1M aqueous solution of lithium hydroxide instead of the 1M aqueous solution of sodium hydroxide. Since the resulting crystals change form due to dissociation of volatile components at a low temperature, it was very difficult to stably supply a product with a constant quality. A
differential scanning calorimeter chart (DSC chart) is shown in Fig. 9, and a X-ray powder diffraction chart is shown in Fig. 10.

### Comparative Example 4

### Crystals of [hemicalcium 4-(azulen-2-ylmethyl)-2-β-D-glucopyranosylphenolate] (1/2 calcium salt of azulene compound A)

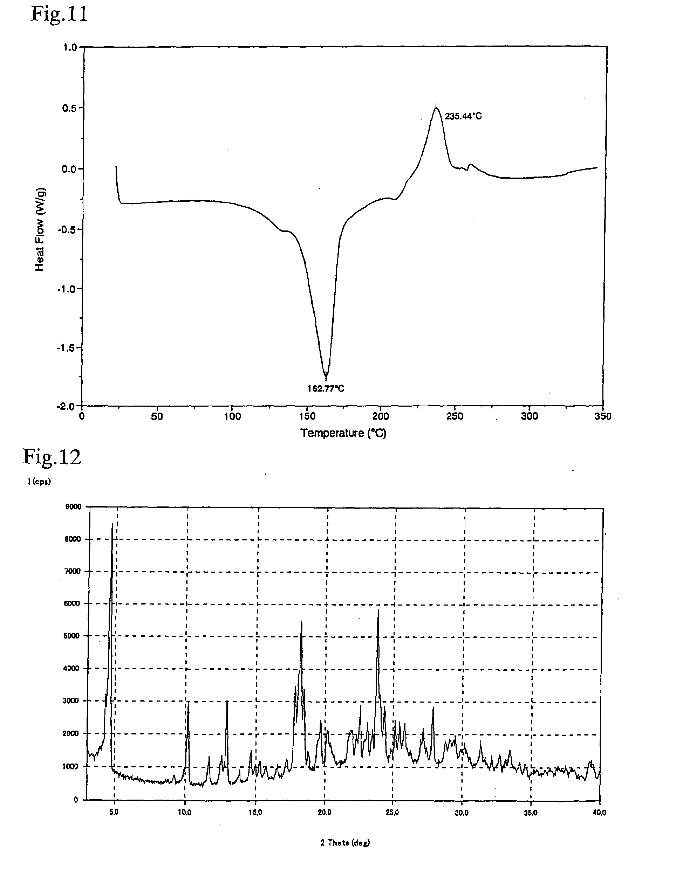

Methanol (3.5 ml) and a 1M aqueous solution of sodium-hydroxide (2.15 ml) were added to [(1S)-1,5-anhydro-1-[5-(azulen-2-ylmethyl)-2-hydroxyphenyl]-D-glucitol (850 mg) and the mixture was stirred at room temperature. The solvent was evaporated under reduced pressure, and the residue was dissolved in water (15 ml). After the addition of a solution of calcium chloride (477 mg) in water (2.5 ml), the mixture was stirred at room temperature. The deposited solid was collected by filtration and washed with water and 2-propanol. The resulting solid was dried with heating under reduced pressure. Tetrahydrofuran was added to the solid, and insoluble components were removed by filtration. The filtrate was concentrated, and the resulting solid was dried with heating under reduced pressure. Dimethylformamide (DMF) (1.0 ml) and water (2.0 ml) were added to the solid (200 mg), and the mixture was heated with stirring until the solid was completely dissolved. After allowing the mixture to cool to room temperature, the deposited crystals were collected by filtration, washed with water, and dried at 60°C under reduced pressure to obtain hemicalcium 4-(azulen-2-ylmethyl)-2-β-D-glucopyranosylphenolate (65 mg). The crystals were obtained only in a form combined with DMF which causes a problem of toxicity. A
differential scanning calorimeter chart (DSC chart) is shown in Fig. 11, and a X-ray powder diffraction chart is shown in Fig. 12.

### Comparative Example 5

### [(1S)-1,5-anhydro-1-[5-(azulen-2-ylmethyl)-2-hydroxyphenyl]-D-glucitol hydrate crystal-1] (hydrate crystal-1 of azulene compound A)

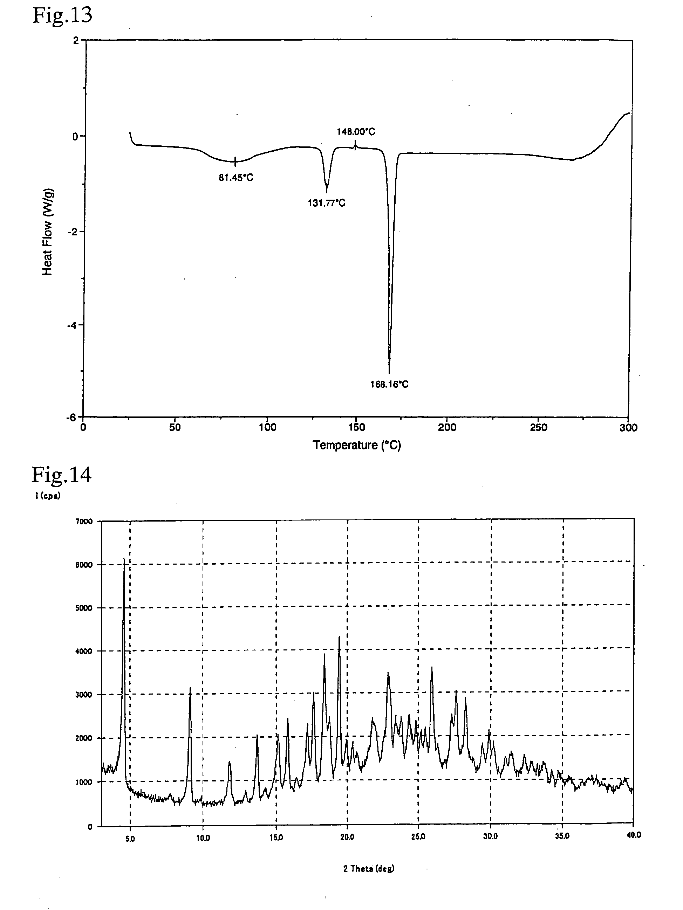

A 1:2 mixture of 2-propanol and water (7.5 ml) was added to (1S)-1,5-anhydro-1-[5-(azulen-2-ylmethyl)-2-hydroxyphenyl]-D-glucitol (300 mg), and the mixture was heated with stirring until the solid was completely dissolved. After allowing the mixture to cool to room temperature, the deposited crystals were collected by filtration, washed with a 1:2 mixture of 2-propanol and water, and dried at 45°C under reduced pressure to obtain hydrate crystal-1 of (1S)-1,5-anhydro-1-[5-(azulen-2-ylmethyl)-2-hydroxyphenyl]-D-glucitol (222 mg). The crystals were dehydrated by heating or drying under reduced pressure and had characteristics of being transformed into anhydride crystal-3 of azulene compound A. However, transformation into anhydride crystal-1 of azulene compounds A and anhydride
crystal-2 of azulene compounds A which takes place in the course of transformation into anhydride crystal-3 differed among lots. Reproducibility was thus not attained by recrystallization. A differential scanning calorimeter chart (DSC chart) is shown in Fig. 13, and a X-ray powder diffraction chart is shown in Fig. 14.

### Comparative Example 6

### [(1S)-1,5-anhydro-1-[5-(azulen-2-ylmethyl)-2-hydroxyphenyl]-D-glucitol hydrate crystal-2] (hydrate crystal-2 of azulene compound A)

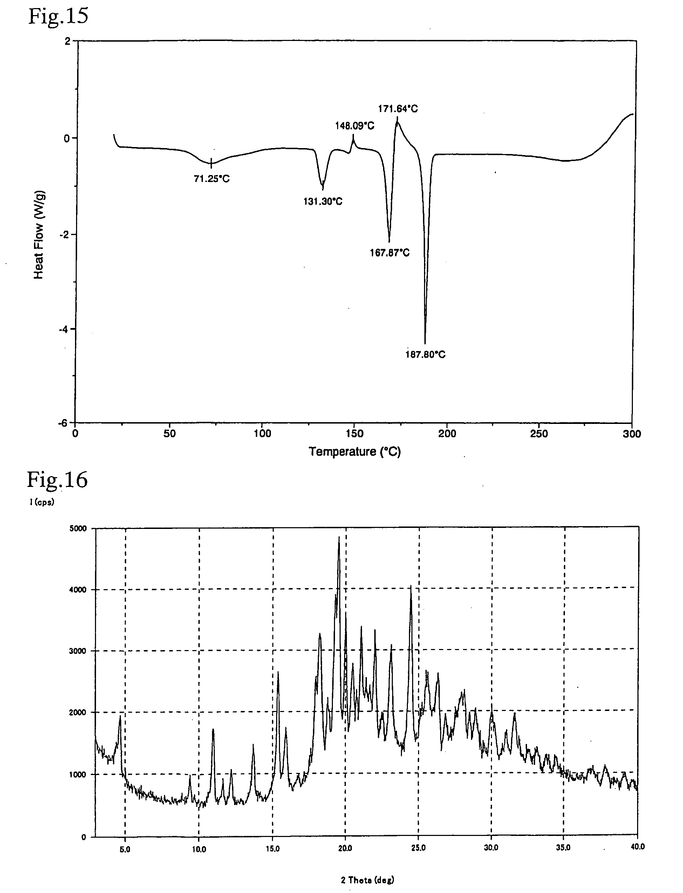

Ethanol (2.0 ml) was added to (1S)-1,5-anhydro-1-[5-(azulen-2-ylmethyl)-2-hydroxyphenyl]-D-glucitol (500 mg), and the mixture was heated with stirring until the solid was completely dissolved. After allowing the mixture to cool to room temperature, the deposited crystals were collected by filtration, washed with ethanol, and dried at 45°C under reduced pressure to obtain hydrate
crystal-2 of (1S)-1,5-anhydro-1-[5-(azulen-2-ylmethyl)-2-hydrophenyl]-D-glucitol (122 mg). The crystal was obtained only in one lot by recrystallization, indicating no reproducibility. A differential scanning calorimeter chart (DSC chart) is shown in Fig. 15, and a X-ray powder diffraction chart is shown in Fig. 16.

### Comparative Example 7

### [(1S)-1,5-anhydro-1-[5-(azulen-2-ylmethyl)-2-hydroxyphenyl]-D-glucitol anhydride crystal-1] (anhydride crystal-1 of azulene compound A)

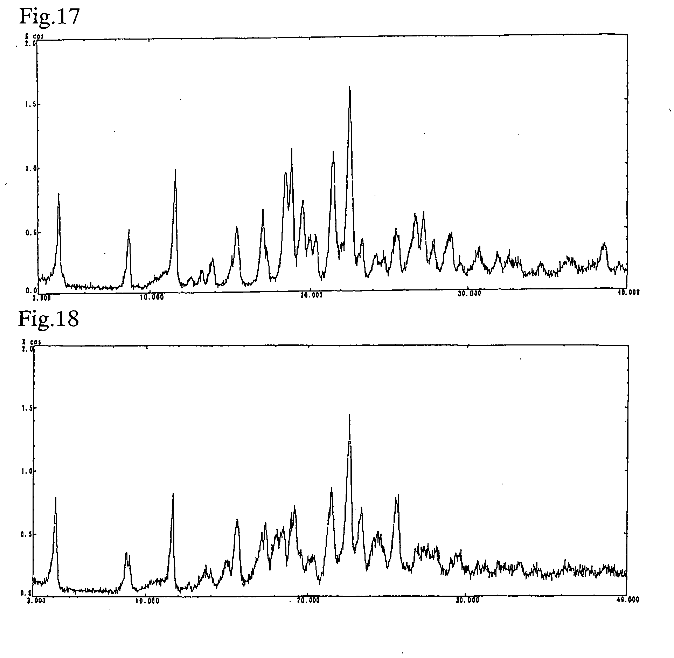

(1S)-1,5-anhydro-1-[5-(azulen-2-ylmethyl)-2-hydroxyphenyl]-D-glucitol hydrate crystal-1 obtained in Comparative Example 5 was placed in an aluminum sampling pan for exclusive use with a differential scanning calorimetry analyzer (DSC analyzer) and heated under a nitrogen atmosphere at a rate of 10°C/min to confirm production of (1S)-1,5-anhydro-1-[5-(azulen-2-ylmethyl)-2-hydroxyphenyl]-D-glucitol anhydride crystal-1 at about 100°C. (1S)-1,5-anhydro-1-[5-(azulen-2-ylmethyl)-2 -hydroxyphenyl]-D-glucitol hydrate crystal-1 obtained in Comparative Example 5 was placed in a copper sampling plate for exclusive use with a heating X-ray powder diffraction apparatus to analyze X-ray powder diffraction in a nitrogen atmosphere at 100°C. Production of (1S)-1,5-anhydro-1-[5-(azulen-2-ylmethyl)-2-hydroxyphenyl]-D-glucitol anhydride crystal-1 was confirmed. This crystal, which is produced by a heat treatment of hydrate crystal-1 of azulene compounds A, is stable only at high temperature and cannot be isolated at room temperature. The X-ray powder diffraction (heating X-ray powder) chart is shown in Fig. 17.

### Comparative Example 8

### [(1S)-1,5-anhydro-1-[5-(azulen-2-ylmethyl)-2-hydroxyphenyl]-D-glucitol anhydride crystal-2] (anhydride crystal-2 of azulene compound A)

(1S)-1,5-anhydro-1-[5-(azulen-2-ylmethyl)-2-hydroxyphenyl]-D-glucitol hydrate crystal-1 obtained in Comparative Example 5 was placed in an aluminum sampling pan for exclusive use with a DSC analyzer and heated in a nitrogen atmosphere at a rate of 10°C/min to confirm production of (1S)-1,5-anhydro-1-[5-(azulen-2-ylmethyl)-2-hydroxyphenyl]-D-glucitol anhydride crystal-2 at about 140°C. (1S)-1,5-anhydro-1-[5-(azulen-2-ylmethyl)- 2-hydroxyphenyl]-D-glucitol hydrate crystal-1 obtained in Comparative Example 5 was placed in a copper sampling plate for exclusive use with a heating X-ray powder diffraction apparatus to analyze X-ray powder diffraction in a nitrogen atmosphere at about 140°C to obtain (1S)-1,5-anhydro-1-[5-(azulen-2-ylmethyl)-2- hydroxyphenyl]-D-glucitol anhydride crystal-2. This crystal, which was produced by a heat treatment of hydrate crystal-1 of azulene compounds A, was stable only at a high temperature and could not be isolated at room temperature. The X-ray powder diffraction (heating X-ray powder) chart is shown in Fig. 18.

### Comparative Example 9

### [(1S)-1,5-anhydro-1-[5-(azulen-2-ylmethyl)-2-hydroxyphenyl]-D-glucitol anhydride crystal-3] (anhydride crystal-3 of azulene compound A)

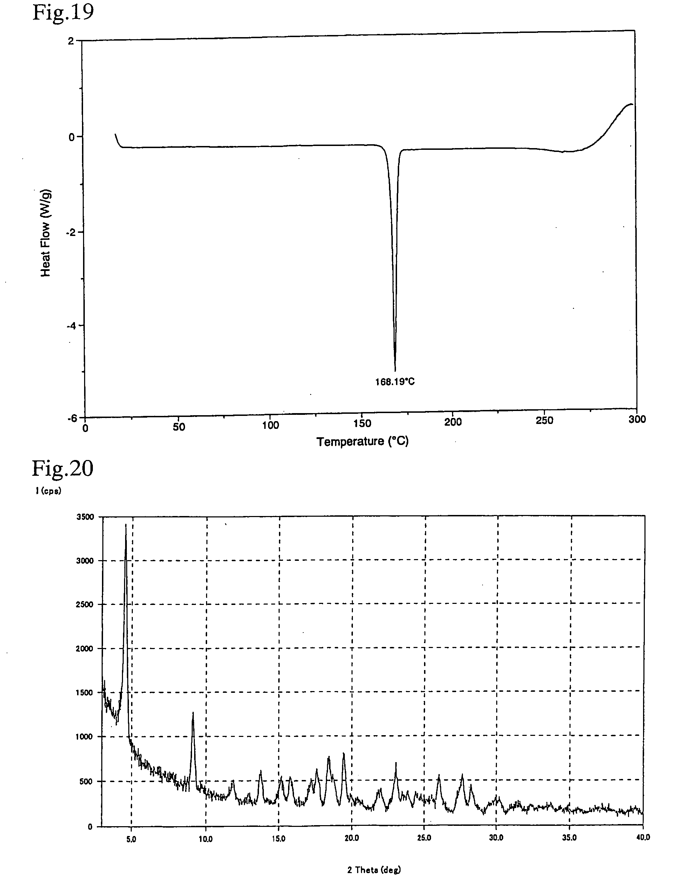

(1S)-1,5-anhydro-1-[5-(azulen-2-ylmethyl)-2-hydroxyphenyl]-D-glucitol hydrate crystal-1 obtained in Comparative Example 5 was placed in an aluminum sampling pan for exclusive use with a differential scanning calorimetry analyzer (DSC analyzer) and heated to 150°C in a nitrogen atmosphere at a rate of 10°C/min to confirm production of (1S)-1,5-anhydro-1-[5-(azulen-2-ylmethyl)-2- hydroxyphenyl]-D-glucitol anhydride crystal-3. This crystal was produced only by a heat treatment of hydrate crystal-1 of azulene compounds A and could not be obtained by recrystallization. A
differential scanning calorimeter chart (DSC chart) is shown in Fig. 19, and a X-ray powder diffraction chart is shown in Fig. 20.

### Comparative Example 10

### [(1S)-1,5-anhydro-1-[5-(azulen-2-ylmethyl)-2-hydroxyphenyl]-D-glucitol anhydride crystal-4] (anhydride crystal-4 of azulene compound A)

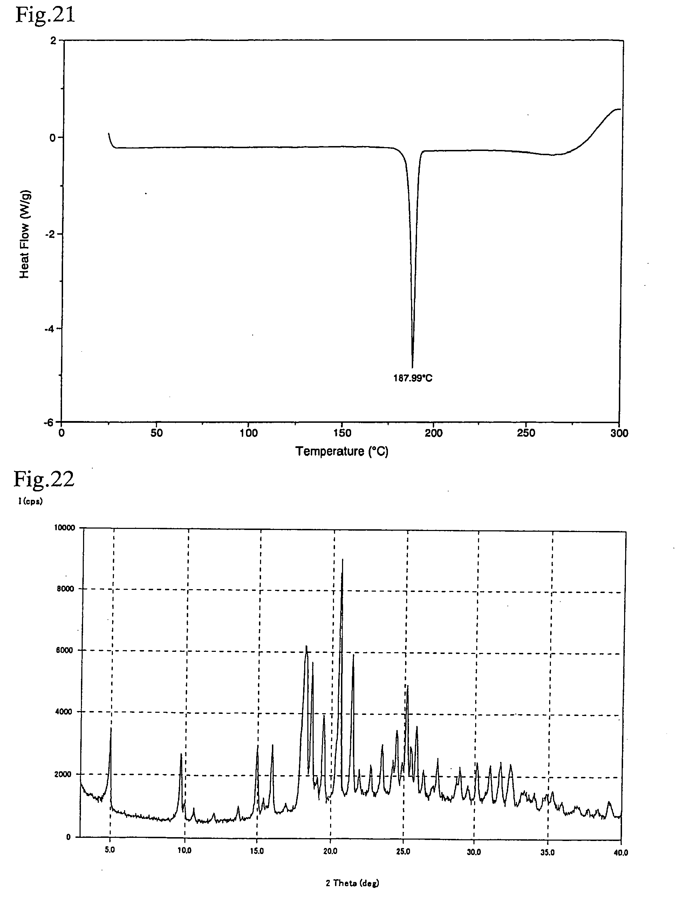

(1S)-1,5-anhydro-1-[5-(azulen-2-ylmethyl)-2-hydroxyphenyl]-D-glucitol hydrate crystal-2 obtained in Comparative Example 6 was placed in an aluminum sampling pan for exclusive use with a differential scanning calorimetry analyzer (DSC analyzer) and heated to 185°C in a nitrogen atmosphere at a rate of 10°C/min to confirm production of (1S)-1,5-anhydro-1-[5-(azulen-2-ylmethyl)- 2-hydroxyphenyl]-D-glucitol anhydride crystal-4. This crystal was produced only by a heat treatment of hydrate crystal-2 of azulene compounds A and could not be obtained by recrystallization. A
differential scanning calorimeter chart (DSC chart) is shown in Fig. 21, and a X-ray powder diffraction chart is shown in Fig. 22.

### Comparative Example 11

### [(1S)-1,5-anhydro-1-[5-(azulen-2-ylmethyl)-2-hydroxyphenyl]-D-glucitol anhydride crystal-5] (anhydride crystal-5 of azulene compound A)

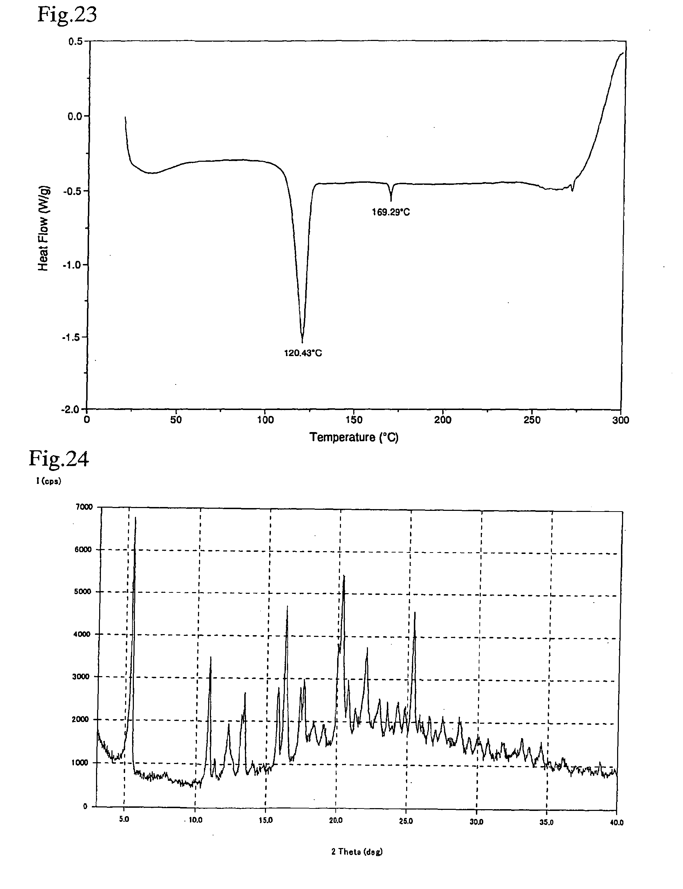

Acetonitrile (3.0 ml) was added to (1S)-1,5-anhydro-1-[5-(azulen-2-ylmethyl)-2-hydroxyphenyl]-D-glucitol (300 mg), and the mixture was heated with stirring until the solid was completely dissolved. After allowing the mixture to cool to room temperature, the deposited crystals were collected by filtration, washed with acetonitrile, and dried at 45°C under reduced pressure to obtain anhydride crystal-5 of (1S)-1,5-anhydro-1-[5-(azulen-2-ylmethyl)-2-hydrophenyl]-D-glucitol (101 mg). The crystal was obtained only in one lot by recrystallization, indicating no reproducibility. Two exothermic peaks were confirmed in differential scanning calorimeter analysis (DSC analysis), suggesting inability to produce crystals as a single crystal form. A
differential scanning calorimeter chart (DSC chart) is shown in Fig. 23, and a X-ray powder diffraction chart is shown in Fig. 24.

### (Storage stability Test)

(2-hydroxyethyl)trimethylammonium-4-(azulene-2-ylmethyl)-2-β-D-glucopyranosylphenolate (46 g) was put into a polyethylene bag, and the opening of the bag was closed with a bead band. The bag was put into another polyethylene bag containing silica gel (12 g). The opening of the bag was closed with a bead band. The bag was placed in a metal can, which was tightly sealed and stored in a dark place at 40°C and 75% RH for six months. Apart from this, (2-hydroxyethyl)trimethylammonium-4-(azulene-2-ylmethyl)-2-β-D-glucopyranosylphenolate (10 g) was put into an open brown container and stored in a dark place at 40°C and 75% RH for six months. The results are shown in Table 3.

**TABLE 3**

| Storage conditions | Storing period (month) | Analogous compounds (%) (HPCL) | | Water content (%) | Quantitative analysis (%) (HPCL) |
|---|---|---|---|---|---|
| | | Maximum of each compound | Total amount | | |
| At the start of storing | 0 | 0.26 | 0.61 | 0.39 | 99.0 |
| 40°C, 75% RH Silica gel/polyethylene bags (double) in a metal can (Anhydride) | 2 | 0.26 | 0.66 | 0.29 | 99.5 |
| | 4 | 0.26 | 0.62 | 0.22 | 99.0 |
| | 6 | 0.27 | 0.44 | 0.19 | 99.5 |
| 40°C, 75% RH Brown glass container Open (Hydrate*) | 2 | 0.25 | 0.58 | 3.78 | 99.2 |
| | 4 | 0.24 | 0.57 | 3.59 | 99.9 |
| | 6 | 0.24 | 0.43 | 3.61 | 98.8 |

| | | | | | |
|---|---|---|---|---|---|
| * The compound was confirmed to change into the hydrate immediately after initiation of storage under the above conditions. | | | | | |

Based on the results shown in Table 3, in which no change was seen in the amount and the quantitative value of the analogous compounds, both anhydride and hydrate of (2-hydroxyethyl)trimethylammonium 4-(azulene-2-ylmethyl)-2-β-D-glucopyranosylphenolate were confirmed to be stable compounds.

From the results of the above Examples and Comparative Examples, it can be seen that the salt of azulene compound A with a base such as sodium, potassium, lithium, or calcium which are commonly used in medicines has a form transformable by dissociation of volatile components even at a low temperature. In addition, crystals of 1/2 Ca salt which can exist only in a DMF-combined form have a problem of toxicity caused by DMF. Thus, these crystals cannot be used as a pharmaceutical.

Furthermore, there are seven types of crystal forms in free-form azulene compound A, that is, hydrate crystal-1, hydrate crystal-2, anhydride crystal-1, anhydride crystal-2, anhydride crystal-3, anhydride crystal-4, and anhydride crystal-5. Some of these crystals are transformed into the other crystal forms, some crystals can be reproduced only with difficulty or can stably exist only at high temperature, involving difficulty in isolating at room temperature, and other crystals can be produced only by a treatment with heat. These crystals can be obtained in some cases but cannot be obtained in other cases, used the same conditions. It is thus difficult to obtain crystals as a single crystal form by controlling production of polymorphs. Therefore, it has been found that a pharmaceutical product cannot be produced from the crystals of free-form azulene compound A.
Differing from crystals of various salts and crystals of free-form azulene compound A, the crystals of the invention can be produced as crystals as a single crystal form with excellent reproducibility, can stably be supplied as a drug substance of a pharmaceutical, and have superior storage stability. Due to this success, the production as a pharmaceutical has been attained for the first time.

### (Pharmacological test)

### [Test for confirming effect of inhibiting activity of Human Na⁺-glucose cotransporter (human SGLT2)]

### 1) Preparation of human SGLT2 expression vector

First, single-stranded cDNAwas reversely transcripted from total RNA originating from human kidney (manufactured by BD Biosciences Clontech) using a Superscript II (manufactured by Invitrogen Corporation) and a random hexamer. Second, using the cDNA as a template, a DNA fragment encoding human SGLT2 (Wells R. G et al., Am. J. Physiol., 1992, 263 (3) F459) was amplified by a PCR reaction using Pyrobest DNA polymerase (manufactured by Takara Bio Inc.) (A primer where a *Hind III* site and an *EcoRI* site were inserted into the 5' side and the 3' side of the DNA fragment, respectively, was used).
The amplified fragment was cloned into a pCR2.1-Topo vector using a Topo TA Cloning Kit (manufactured by Invitrogen Corporation), and the cloned vector was transfected into a competent cell of *Escherichia coli* JM109. Ampicillin-resistant clones were cultured in a LB medium containing ampicillin (100 mg/l). A plasmid was purified from the cultured *Escherichia coli* using the method of Hanahan (see Maniatis et al., "Molecular Cloning"). A DNA fragment for encoding a human SGLT2 was obtained by the *Hind III*/EcoRI digestion of the plasmid and ligated and cloned to the same site of the expression vector pcDNA3.1 (manufactured by Invitrogen Corporation) using a T4 DNA ligase (manufactured by Roche Diagnostics). The ligated clone was transfected into a competent cell of *Escherichia coli* JM109 in the same manner as described above and cultured in an LB medium containing ampicillin, and a human SGLT2 expression vector was obtained using the method of Hanahan.

### 2) Preparation of human SGLT2 expression cells

The human SGLT2 expression vector was transfected into a CHO-K1 cells using Lipofectamine 2000 (manufactured by Invitrogen Corporation). The cell was cultured in a Ham's F12 medium (manufactured by Nissui Pharmaceutical Co., Ltd.) containing Penicillin (50 IU/ml, manufactured by Dainippon Pharmaceutical Co., Ltd.), streptomycin (50 µg/ml, manufactured by Dainippon Pharmaceutical Co., Ltd.), Geneticin (40 µg/ml, manufactured by Invitrogen Corporation), and 10% fetal bovine serum in the presence of 5% CO₂ at 37°C for two weeks, and Geneticin-resistant clones were obtained. A cell which stably expresses the human SGLT2, which exhibits sodium-dependent intake of methyl-α-D-glucopyranoside, was obtained from among these clones (See the following paragraphs for the method for measuring the methyl-α-D-glucopyranoside intake).

### 3) Measurement of inhibition of methyl-α-D-glucopyranoside intake

After removing the medium of a CHO cell which stably express the human SGLT2, a pretreatment buffer solution (buffer solution of pH 7.4 containing choline chloride (140 mM), potassium chloride (2 mM), calcium chloride (1 mM), magnesium chloride (1 mM), 2-[4-(2-hydroxyethyl)-1-piperazinyl]ethanesulfonic acid (10 mM), and tris(hydroxymethyl)aminomethane (5 mM)) was added in the amount of 100 µl per well, and incubated at 37°C for 20 minutes.
11 µl of methyl-α-D-(U-14C) glucopyranoside (manufactured by Amersham Pharmacia Biotech) was added to and mixed with 1,000 µl of a buffer solution for intake containing a test crystal (buffer solution of pH 7.4 containing sodim chloride (140 mM), potassium chloride (2 mM), calcium chloride (1 mM), magnesium chloride (1 mM), methyl-α-D-glucopyranoside (50 µM),
2-[4-(2-hydroxyethyl)-1-piperazinyl]ethanesulfonic acid (10 mM), and tris(hydroxymethyl)aminomethane (5 mM)) to prepare a buffer solution for intake. A buffer solution for intake without a test crystal was prepared for a control group. A
buffer solution for basal intake without a test crystal containing choline chloride (140 mM) instead of sodium chloride for measuring the basal intake in the absence of sodium was prepared as well.

After removing the pretreatment buffer solution, the buffer solution for intake was added (25 µl per well) and incubated at 37°C for two hours. After removing the buffer solution for intake, a buffer solution for washing (buffer solution of pH 7.4 containing choline chloride (140 mM), potassium chloride (2 mM), calcium chloride (1 mM), magnesium chloride (1 mM), methyl-α-D-glucopyranoside (10 mM), 2-[4-(2-hydroxyethyl)-1-piperazinyl]ethanesulfonic acid (10 mM), and tris(hydroxymethyl)aminomethane (5 mM)) was added (200 µl per one well). The mixture was immediately removed. This washing operation was carried out once more. 0.5% lauryl sodium sulfate was added (25 µl per well) to solubilize the cells. 75 µl of Microscint 40 (manufactured by PerkinElmer, Inc.) was added to the solubilized cell, and the radiation activity was measured using a microscintillation counter TopCount (manufactured by PerkinElmer, Inc.). The value obtained by subtracting the basal intake amount from the intake amount of the control group was defined as 100%. The concentration for 50% inhibition of the above value (IC₅₀ value) was calculated from a concentration-inhibition curve using the least-squares method. As a result, the choline salt of azulene compound A shown in Example 1 and the choline salt hydrate of azulene compound A shown in Example 2 showed values equivalent to the value (8.9 nM) shown in Example 75 of Table 24 of Patent Document 1.

### INDUSTRIAL APPLICABILITY

Since the crystals of the present invention have excellent storage stability and exhibit Human Na⁺-glucose cotransporter-inhibiting action and antihyperglycemic action, the crystals useful as a pharmaceutical, particularly as a diabetes-treating medicine for treating and preventing insulin-dependent diabetes mellitus (type-1 diabetes), non-insulin-dependent diabetes mellitus (type-2 diabetes), insulin resistance diseases, and overweight.

The excellent storage stability and the superior Human Na⁺-glucose cotransporter-inhibiting action and antihyperglycemic action of the crystals of the present invention have been confirmed by the above storage stability test and the pharmacological test.

## Claims

1. A choline salt of (1S)-1,5-anhydro-1-[5-(azulen-2-ylmethyl)-2-hydroxyphenyl]-D-glucitol.

2. A choline salt crystal of (1S)-1,5-anhydro-1-[5-(azulen-2-ylmethyl)-2-hydroxyphenyl]-D-glucitol having an endothermic peak at 194 to 198°C measured by differential scanning calorimetry (DSC analysis).

3. A choline salt crystal of (1S)-1,5-anhydro-1-[5-(azulen-2-ylmethyl)-2-hydroxyphenyl]-D-glucitol having main peaks at about 2θ (°) of 5.58, 14.72, 16.80, 17.82, 21.02, and 22.46 measured by X-ray powder diffraction.

4. A choline salt crystal of (1S)-1,5-anhydro-1-[5-(azulen-2-ylmethyl)-2-hydroxyphenyl]-D-glucitol having an endothermic peak at 194 to 198°C measured by differential scanning calorimetry (DSC analysis) and main peaks at about 2θ (°) of 5.58, 14.72, 16.80, 17.82, 21.02, and 22.46 measured by X-ray powder diffraction.

5. A choline salt hydrate crystal of (1S)-1,5-anhydro-1-[5-(azulen-2-ylmethyl)-2-hydroxyphenyl]-D-glucitol having a broad endothermic peak at about 78°C and an endothermic peak at 195 to 199°C measured by differential scanning calorimetry (DSC analysis).

6. A choline salt hydrate crystal of (1S)-1,5-anhydro-1-[5-(azulen-2-ylmethyl)-2-hydroxyphenyl]-D-glucitol having main peaks at about 2θ (°) of 5.66, 17.08, 17.66, 19.02, 19.58, and 22.14 measured by X-ray powder diffraction.

7. A choline salt hydrate crystal of (1S)-1,5-anhydro-1-[5-(azulen-2-ylmethyl)-2-hydroxyphenyl]-D-glucitol having a broad endothermic peak at about 78°C and an endothermic peak at 195 to 199°C measured by differential scanning calorimetry (DSC analysis) and main peaks at about 2θ (°) of 5.66, 17.08, 17.66, 19.02, 19.58, and 22.14 measured by X-ray powder diffraction.

8. A pharmaceutical composition comprising a choline salt according to claim 1, a choline salt crystal according to any one of claims 2 to 4, or a choline salt hydrate crystal according to any one of claims 5 to 7 as an effective ingredient.

9. The pharmaceutical composition according to claim 8, further comprising a pharmaceutically acceptable excipient.

10. The pharmaceutical composition according to claim 8 or claim 9, which is a diabetes treating agent.
